# EUROPÄISCHE PATENTSCHRIFT

(11) **EP 3 044 583 B1**
(45) Veröffentlichungstag und Bekanntmachung des Hinweises auf die Patenterteilung: **12.02.2020**
(21) Anmeldenummer: 14766677.0
(22) Anmeldetag: 09.09.2014
(51) Int. Cl.: G01N 33/14

(54) **VERFAHREN ZUR HALTBARKEITSUNTERSUCHUNG BEI VERPACKUNGEN**
METHOD FOR INVESTIGATING THE SHELF LIFE ON PACKAGING
PROCÉDÉ DE TEST DE LA CONSERVABILITÉ D'EMBALLAGES

(30) Priorität: 10.09.2013 DE 102013109945
(43) Veröffentlichungstag der Anmeldung: 20.07.2016
(73) Patentinhaber: Steinfurth Mess-Systeme GmbH, 45309 Essen (DE)
(72) Erfinder: ANGRES, Johann, 44803 Bochum (DE)
(74) Vertreter: Vogel, Andreas
(86) Internationale Anmeldenummer: PCT/EP2014/069188
(87) Internationale Veröffentlichungsnummer: WO 2015/036399

(56) Entgegenhaltungen:
- EP-A2- 1 626 275
- US-A- 5 473 161
- US-B1- 6 964 191
- EUGENIO IVORRA ET AL: "Detection of expired vacuum-packed smoked salmon based on PLS-DA method using hyperspectral images", JOURNAL OF FOOD ENGINEERING, Bd. 117, Nr. 3, 1. August 2013 (2013-08-01), Seiten 342-349, XP055132038, ISSN: 0260-8774, DOI: 10.1016/j.jfoodeng.2013.02.022
- KOENIG R: "ZUR BESCHR[NKUNG DES ANSPRUCHSINHALTS DURCHBAR-DERIVATE", MITTEILUNGEN DER DEUTSCHEN PATENTANWAELTE, HEYMANN, KOLN, DE, 1. Januar 1997 (1997-01-01), Seite 62, XP001248632, ISSN: 0026-6884

## Beschreibung

Die vorliegende Erfindung ist auf ein Verfahren zur Haltbarkeitsuntersuchung von Lebensmitteln in Verpackung gemäß des Oberbegriffes von Anspruch 1 gerichtet. Des Weiteren betrifft die vorliegende Erfindung auch eine Vorrichtung zur Haltbarkeitsuntersuchung von Lebensmitteln in Verpackungen gemäß des Oberbegriffes von Anspruch 12. Dabei kann eine derartige Vorrichtung eine mechanische Aufnahme für die Verpackung und ein Meßkopf sowie eine Auswerteeinheit aufweisen.

Aus dem Stand der Technik sind diverse Verfahren zur Haltbarkeitsuntersuchung von Lebensmitteln in Verpackungen bekannt. Derartige Verfahren betreffen z. B. jede Form von Getränken sowie Milchprodukte, in Form von Käse, Joghurt sowie Fleischwaren, Süßigkeiten, Gewürze und dergleichen. Dabei gibt das Mindesthaltbarkeitsdatum (kurz MHD genannt) von den Lebensmittelherstellern zu ihren Lebensmitteln an, bis zu welchem Termin das Lebensmittel bei sachgerechter Aufbewahrung (insbesondere Einhaltung der im Zusammenhang mit dem Mindesthaltbarkeitsdatum genannten Lagertemperatur) auf jeden Fall ohne wesentliche Geschmacks- und Qualitätseinbußen sowie gesundheitliches Risiko zu konsumieren ist. Bei dem Mindesthaltbarkeitsdatum handelt es sich nicht um ein Verfallsdatum, da i. d. R. die Lebensmittel auch nach dem angegebenen Mindesthaltbarkeitsdatum, noch verzehrfähig sind.

Die Lebensmittelhersteller verwahren von jeder Herstellungsserie von Lebensmittel Proben auf, anhand derer sie die Qualität der Serie von Lebensmittelprodukte nachweisen können, um somit möglichen Regressforderungen von Konsumenten rechtlich sicher im Gegenbeweis begegnen zu können. Außerdem werden hier die einzelnen Proben von Lebensmitteln aus einer Serie zu vorgegebenen Zeitpunkten auf ihre Qualität hin überprüft. Sollte dabei das Ergebnis - gegen alle Erwartungen - negativ ausfallen, so kann der Lebensmittelhersteller sofort die gesamte Serie durch z. B. einen öffentlichen Aufruf aus dem Verkehr ziehen. Da jedoch i. d. R. bei der entsprechenden Probenmessung die Qualität ausreichend ist, reicht es, wenn der Lebensmittelhersteller die Ergebnisse schriftlich dokumentiert und aufbewahrt. Die bereits untersuchten Proben von Lebensmitteln sind jedoch i. d. R. nach der Untersuchung unbrauchbar, da die Verpackung geöffnet worden sind und somit Luft an die Lebensmittel gelangen konnte. Außerdem wird i. d. R. durch die Untersuchung auch die Aufbewahrungstemperatur verändert, was ebenfalls nicht gewünscht ist und sich auf das Lebensmittel negativ auswirken kann. Aus diesem Grund muss der Lebensmittelhersteller entsprechend viele Proben von Lebensmitteln aufbewahren, um zu den jeweiligen Zeitpunkten eine zerstörende Probemessung vornehmen zu können. Hierdurch ist es notwendig, dass der Lebensmittelhersteller entsprechend viele Proben aus einer Serie von Lebensmitteln für spätere Untersuchungen bevorraten muss. Will z. B. der Lebensmittelhersteller über die gesamte Zeit der Mindesthaltbarkeitsdauer einer Serie von Lebensmittelprodukten den Qualitätsnachweis führen, muss er z. B. bei x unterschiedlichen Zeiträumen je m-Proben untersuchen, so benötigt er insgesamt x * m-Proben (Produkt von x und m = n_{ges}-Proben), die er aus einer Serie zurückhalten und aufbewahren muss. Üblicherweise werden zu einem Zeitpunkt mindestens zwei oder drei Proben (m = 2 oder 3) untersucht, um eine statistische Sicherheit zu erlangen. Somit ist der Aufbewahrungs- und Bevorratungsbedarf sowie die dadurch entstehenden Verluste bei der Lebensmittelproduktion sehr groß.

Bei Lebensmitteln in Form von Getränken hat sich herausgestellt, dass der vorhandene Druck in der Flasche sowie das Material der Flasche, beispielsweise Glas, Porzellan oder PET maßgeblich für die Qualität und Geschmack der Getränke ist.

US 6964191 offenbart ein artverwandtes Verfahren sowie eine entsprechende Vorrichtung zur Haltbarkeitsuntersuchung von Lebensmitteln in Verpackungen.

Aufgabe der vorliegenden Erfindung ist es somit die zuvor beschriebenen Nachteile aus dem Stand der Technik zu überwinden. Insbesondere ist es Aufgabe der vorliegenden Erfindung ein Verfahren zur Haltbarkeitsuntersuchung von Lebensmitteln in Verpackungen bereitzustellen, welches kostengünstig durchzuführen ist und bei dem die Gesamtanzahl (n_{ges}= x * m) der zu bevorratenden Lebensmittel einer Serie möglichst reduziert ist. Ebenfalls ist es eine zusätzliche Aufgabe der vorliegenden Erfindung auch eine Vorrichtung zur Haltbarkeitsuntersuchung von Lebensmitteln in Verpackungen bereitzustellen, mit deren Hilfe die Nachteile aus dem Stand der Technik zumindest teilweise überwunden werden.

Die vorliegende Aufgabe wird durch ein erfindungsgemäßes Verfahren zu Haltbarkeitsuntersuchung von Lebensmitteln in Verpackungen mit den Merkmalen des Anspruches 1, insbesondere aus dem kennzeichnenden Teil, gelöst. Ebenfalls wird zur Lösung der Aufgabe eine Vorrichtung mit den Merkmalen des Anspruches 12 vorgeschlagen. In den abhängigen Verfahrens- und Vorrichtungsansprüche sind bevorzugte Weiterbildungen der Erfindung aufgeführt. Merkmale, die zu dem erfindungsgemäßen Verfahren offenbart werden, gelten auch für die erfindungsgemäße Vorrichtung und umgekehrt. Außerdem kann das erfindungsgemäße Verfahren auf der erfindungsgemäßen Vorrichtung durchgeführt werden.

Im vorliegenden Text werden die Begriffe "Lebensmittel" und "Verpackungen" erwähnt, womit gemeint ist, dass es sich um die gleichen Lebensmittel handelt und diese aus einer Herstellungsserie, die zu einem bestimmten Zeitpunkt gefertigt worden sind, stammen. Auch sind diese Lebensmittel in den gleichen Verpackungen angeordnet. Unter dem Begriff "Probe" wird das zu untersuchende Lebensmittel in der Verpackung verstanden. Die Buchstaben "RM" stehen im folgenden Text für eine Referenzmessung. Der Buchstabe "M" hingegen steht im folgenden Text für eine normale (Gebrauchs-) Messung. Die beiden Indizes "d" und "i" stehen für eine direkt und indirekt Messung.

Das erfindungsgemäße Verfahren zur Haltbarkeitsuntersuchung von Lebensmitteln in Verpackungen weist die zumindest die folgenden Schritte auf:
a) Erstellen zumindest einer **Referenzmessung** mit:
   a1) einer direkten und indirekten Probenmessung RMd,i zum Zeitpunkt Tx
   a2) Wiederholung der direkten und indirekten Probenmessung RMd,i in vordefinierten Zeitabständen Δt
   a3) Speicherung der Referenzmeßergebnisse RMd,i der Probenmessungen
b) Erstellen einer **Vergleichsmessung** mit:
   b1) zumindest einer indirekten Probenmessung Mi zum Zeitpunkt Tx
   b2) Vergleich des indirekten Meßergebnisses Mi mit dem entsprechenden Referenzmeßergebnis RMi vom Vergleichszeitpunkt Tx
   b3) Durchführung von zumindest einer direkten Probenmessung Md zum Zeitpunkt Tx bei einer überschrittenen Abweichung des Vergleichsergebnisses aus Schritt b2)
   b4) Wiederholung der indirekten Probenmessung in vordefinierten Zeitabständen Δt
   b5) Speicherung der Meßergebnisse Md,i der Probenmessungen

Dabei ist zu bemerken, dass zumindest beim ersten Mal der Schritt a) zur Referenzmessung vor dem Schritt b) der Vergleichsmessung durchgeführt werden muss. Dabei empfiehlt sich für jedes Lebensmittelprodukt (gemeint ist das gleiche Lebensmittel, z.B. alkoholfreies Bier einer bestimmten Marke) eine eigene Referenzmessung nach Schritt a), um somit exakte Referenzmessungen für die spätere Vergleichsmessung zu erhalten. Idealerweise findet nicht nur eine Referenzmessung nach Schritt a) statt, sondern zwei oder drei, anhand derer man feststellen kann, inwieweit schon die einzelnen Referenzmessungen voneinander abweichen. Sollte sich dabei herausstellen, dass eben keine Abweichung vorliegt, so kann zukünftig bei gleichen Lebensmitteln die Anzahl der Referenzmessungen minimiert werden, sodass idealerweise nur eine Referenzmessung gemäß Schritt a) durchgeführt werden muss. Des Weiteren werden bei der Referenzmessung auch weitere Voraussetzungen festgelegt, wie z. B. die vordefinierten Zeitabstände Δt der einzelnen Probemessungen.

Hierbei kann z. B. die Mindesthaltbarkeitsdauer durch die maximal gewünschte Anzahl der Probemessungen beim Zeitpunkt Tx geteilt werden, woraus sich dann der vordefinierte Zeitabstand Δt ergibt. Beispielsweise kann bei einer Mindesthaltbarkeitsdauer von 12 Monaten gewünscht sein, nur 12 zeitlich unterschiedliche Probenmessungen durchzuführen. In diesem Fall beträgt dann der vordefinierte Zeitabstand Δt genau einen Monat. Bei der Referenzmessung im Messschritt a1) empfiehlt es sich, zuerst eine indirekte Probenmessung RMi zum Zeitpunkt Tx durchzuführen und dann die direkte Probenmessung RMd zum selben Zeitpunkt Tx. Dabei kommt es nicht darauf an, dass es sich der Zeitpunkt Tx um wenige Minuten variiert, da üblicherweise das Mindesthalbarkeitsdatum zumeist Wochen, Monate oder sogar Jahre beträgt. Wichtig ist jedoch, dass die Voraussetzung für die indirekte und direkte Probenmessung, wie z. B. die Temperatur der Probe (des Lebensmittels) gleich ist und das Lebensmittel aus einer Serie mit dem gleichen Herstellungsdatum stammt. Auch kann im Schritt a1) mehrfach eine indirekte und direkte Probemessung RMd,i zum Zeitpunk Tx an einer Probe stattfinden, um somit Messfehler und Messtoleranzen ausschließen zu können. Im Schritt a2) findet dann wieder die direkte und indirekte Probenmessung RMd,i zum Zeitpunkt Tx+1, bzw. nach einem vordefinierten Zeitabstand Δt statt. Hierbei werden wieder ausschließlich die Lebensmittel aus der gleichen Serie (zu Schritt a1)) für die erneute Probenmessung verwendet. Dabei kann die Messung selbst wie in Schritt a1), allerdings zum jüngeren Zeitpunkt Tx+1 vonstatten gehen. Auch versteht es sich von selbst, dass bei der direkten Probemessung i. d. R. nur Lebensmittel einer permanent geschlossenen Verpackung zur Messung herangezogen werden können. Somit scheiden Lebensmittelverpackungen aus, die zuvor bei einer Messung im Messchritt a1) im Rahmen der direkten Probenmessung zerstört worden sind, bzw. deren Verpackungen geöffnet worden sind. Auch in Schritt a2) kann jede Probe mehrfach einer direkten oder indirekten Probenmessung RMd,i unterzogen werden. In Schritt a3) werden die Referenzmessergebnisse RMd,i aus den Schritten a1) und a2) gespeichert und archiviert. Auch ist es denkbar, dass bereits eine statistische Auswertung der Referenzmessergebnisse stattfindet (beispielsweise Mittelwertsbildung), z. B. wenn von ein und derselben Probe zum gleichen Zeitpunkt Tx mehrfache Probenmessungen direkt und indirekt durchgeführt worden sind. Außerdem kann auch statistisch versucht werden, zu erfassen, inwieweit sich die Probenmessungen RMd,i von Zeitpunkt Tx zum Zeitpunkt Tx+1 oder weiteren Zeitpunkten Txy verändern.

Im Schritt b) findet dann die eigentliche Vergleichsmessung an den gleichen Lebensmittelprodukten (gemeint ist z. B. alkoholfreies Bier der Brauererei X von der Marke Y, oder koffeinhaltige Limonade mit Zucker der Marke Z) statt. Hierbei können sich die Schritte a) und b) zumindest zeitlich teilweise überlappen, d. h. es muss keine vollständige Referenzmessung gemäß Schritt a) über die gesamte Mindesthaltbarkeitsdauer durchgeführt werden, um bereits mit einer ersten Vergleichsmessung gemäß Schritt b) zu beginnen. Vielmehr reicht es aus, wenn die ersten Referenzmessergebnisse RMd,i zum Zeitpunkt Tx aus Schritt a1) vorliegen, bevor mit Schritt b) der Vergleichsmessung begonnen wird. Im Schritt b1) wird zumindest eine indirekte Probenmessung Mi zum Zeitpunkt Tx am selben Lebensmittel aus der Referenzmessung gemäß Schritt a) durchgeführt. Hierbei kann ebenfalls die indirekte Probenmessung mehrfach wiederholt werden, um Messfehler auszuschließen. Dabei soll der Schritt b1) unter den gleichen Messvoraussetzungen der Messung Mi bei Schritt a1) durchgeführt werden, worunter gleiche Untersuchungsbedingungen verstanden wird, wie z. B. Messtemperatur, Umgebungsdruck, Luftfeuchtigkeit und dergleichen. Im Schritt b2) werden dann die unter b1) gemessenen indirekten Messergebnisse Mi mit den entsprechenden Referenzmessergebnissen RMi zum Vergleichszeitpunkt Tx verglichen. Sollte hierbei eine deutliche Abweichung, z. B. durch eine Differenz der Messergebnisse zwischen den Referenzmessergebnissen und den Vergleichsmessergebnissen aus demselben Zeitpunkt Tx vorliegen, so ist auch eine direkte Probenmessung Md idealerweise an derselben Verpackung des Lebensmittels, mit der die indirekte Probenmessung durchgeführt worden ist, erforderlich. Sofern jedoch keine überschrittene Abweichungen zwischen den Referenzmessergebnissen RMd,i und der direkten Probenmessung Md,i zum Zeitpunkt Tx vorliegt, kann auf eine direkte Probemessung zum Zeitpunkt Tx verzichtet werden. Um eventuell eine größere Messsicherheit zu erhalten, kann jedoch auch bei der Vergleichsmessung gemäß Schritt b) immer zumindest eine direkte Probenmessung Md zum Zeitpunkt Tx durchgeführt werden, auch wenn keine Abweichungen zwischen den Referenz und den Vergleichsergebnissen vorliegt. Im Schritt b4) findet eine Wiederholung der direkten Probemessungen in vordefinierten Zeitabständen Δt statt. Der Schritt b4) erfolgt somit zweckmäßigerweise, erst wenn keine gemessene Abweichungen gemäß Schritt b3) ermittelt werden konnte. Der Schritt b4) kann auch an ein und derselben Probe, bzw. Lebensmittel zum Zeitpunkt Tx durchgeführt werden, um eine höhere statistische Genauigkeit zu erlangen. Hierdurch kommen die Vorteile des erfindungsgemäßen Verfahrens zum Vorschein, da die Proben eben bei der indirekten Messung unversehrt bleiben und für weitere Messungen zur Verfügung stehen. Somit lässt sich die benötigte Probenzahl n eindeutig in der laufenden Überwachung deutlich reduzieren. Damit lassen sich Herstellungsverluste und Aufbewahrungskosten sowie Entsorgungskosten für die erforderlichen Proben deutlich reduzieren. Im Schritt b5) werden die erhaltenen Messergebnisse Md,i aus den bereits durchgeführten Schritten b1) bis b4) gespeichert und für Nachweiszwecke aufbewahrt.

Das vorliegende Verfahren kann für unterschiedliche Lebensmittel in unterschiedlichen Verpackungen durchgeführt werden, wobei jedoch für jedes Lebensmittel in seiner jeweiligen Verpackung das erfindungsgemäße Verfahren durchgeführt werden sollte, um zumindest exakte Referenzmessungen gemäß Schritt a) zu erhalten.

Erfindungsgemäß kann es vorgesehen sein, dass eine direkte Probenmessung des Lebensmittels durch Zerstörung der Verpackung stattfindet. Hierbei ist es denkbar, dass die Verpackung z. B. durch ein Anstechmittel angestochen wird, um wenigstens einen Meßsensor in die Verpackung einführen zu können. Durch diesen Meßsensor ist es dann möglich, zumindest eine physikalische, chemische und/oder biologische Eigenschaft des Lebensmittels zu messen. Hierbei kann es sich z. B. um die Temperatur, den Druck, die Feuchtigkeit, den elektrischen Widerstand oder weitere Eigenschaften des Lebensmittels handeln, die direkt messbar sind. Die direkte Probenmessung weist damit den Vorteil auf, dass die Messergebnisse von dem Meßsensor unter Ausschluss der Messumgebung und somit ohne Messfehler stattfindet. Damit kann eine exakte Probenmessung stattfinden, die eine hohe Genauigkeit der gemessenen Eigenschaft des Lebensmittels aufweist. Allerdings weist die direkte Probenmessung auch den Nachteil auf, dass die Verpackung i. d. R. nicht wieder verschlossen werden kann, sodass am Ende der Probenmessung, die jedoch zum gleichen Zeitpunkt Tx auch mehrfach ausgeführt werden kann, das Lebensmittel mit der Verpackung unbrauchbar, insbesondere für später Messungen ist. Dies liegt einerseits daran, dass nach der Messung Luft in die Verpackung eindringt und andererseits, dass z. B. ein Überdruck oder ein Inertgas nach der direkten Probemessung entweichen kann. Außerdem wird die Kühlkette der gemessenen Lebensmittelprobe durch die Messung i. d. R. unterbrochen.

Ferner ist es im Rahmen der Erfindung denkbar, dass eine indirekte Probenmessung des Lebensmittels stattfindet, bei der eine zerstörungsfreie Untersuchung vorgenommen wird. Hierbei bleibt die Verpackung i. d. R. unversehrt, da eben die Probenmessungen ausschließlich durch die verschlossene Verpackung stattfinden. Dabei können insbesondere physikalische, chemische und/oder biologische Eigenschaften des Lebensmittels vorzugsweise kontaktlos (zum Lebensmittel) durch die Verpackung gemessen werden. Dieses kann z. B. durch optische, induktive, kapazitive und/oder elektromagnetische Messungen usw. erfolgen. Dabei können auch Röntgen- oder Ultraschallmessungen sowie magnetische Resonanzmessungen oder dergleichen zum Einsatz kommen.

Bei dem erfindungsgemäßen Verfahren kann es ferner vorgesehen sein, dass bei einer Probemessung zumindest Temperatur- oder der Druck in der Verpackung gemessen wird. Durch die zuvor genannten und gemessenen Eigenschaften und ggf. die weiteren physikalischen, chemischen und/oder biologischen Eigenschaften des Lebensmittels lässt sich dann eine Folgerung über die Qualität und den Geschmack des Lebensmittels und somit über das Mindesthaltbarkeitsdatum erzielen.

Anhand der Referenzmessungen gemäß Schritt a) ist es grundsätzlich bekannt, wie sich die physikalischen, chemischen und/oder biologischen Eigenschaften des Lebensmittels im Laufe der Zeit verändern und eine Grenze erreicht ist, bei der die Qualität und der Geschmack des Lebensmittels nicht mehr ausreichend ist, um den Vorstellungen der Lebensmittelherstellern zu genügen. Da zumindest eine der zuvor genannten Eigenschaften des Lebensmittels sich über die Zeit des Mindesthaltbarkeitsdatums wesentlich verändert, ist damit auch eine exakte Messung der Qualität und des Geschmacks des Lebensmittels über die Zeit möglich.

Ferner ist es im Rahmen der Erfindung denkbar, dass ein CO₂ Gehalt in der Verpackung aus den beiden Messwerten der Temperatur und des Druckes ermittelt wird. Hierzu kann insbesondere eine Berechnungsfunktion dienen, die als Eingangswerte zumindest die Temperatur und den Druck in einer Verpackung benötigen und dann anhand dieser Eingangswerte den CO₂ Gehalt in dem entsprechenden Lebensmittel bestimmen. Üblicherweise wird für jedes Lebensmittel (z. B. Bier, Limonade usw.) eine entsprechende Berechnungsfunktion in Abhängigkeit von Temperatur und Druck ermittelt, mit deren Hilfe sich dann der entsprechende CO₂ Gehalt in der Verpackung ermitteln lässt. Gerade bei Getränken, die im Flüssigkeitsbehälter oder Flaschen angeordnet sind, spielt der CO₂ Gehalt eine wesentliche Rolle.

Im Rahmen der Erfindung ist es optional vorgesehen, dass der CO₂ Gehalt zumindest in der Verpackung oder im Lebensmittel die Haltbarkeit wesentlich beeinflusst. Hierdurch wird durch den CO₂ Gehalt auch das Mindesthaltbarkeitsdatum im Wesentlichen beeinflusst. Sollte sich somit der CO₂ Gehalt im Laufe der Zeit in der Verpackung reduzieren, da z. B. ein Teil durch die Verpackung diffundiert, so nimmt auch die Haltbarkeit des Lebensmittels entsprechend über die Zeit ab. Damit ist es vorzugsweise notwendig, den CO₂ Gehalt in der Verpackung und/oder in dem Lebensmittel regelmäßig in zeitlichen Abständen, beispielsweise in vordefinierten Zeitabständen Δt zu kontrollieren.

Bei den zuvor erwähnten Lebensmitteln kann es sich insbesondere um ein Getränk handeln, damit sind flüssige Lebensmittel gemeint. Hierbei ist es denkbar, dass das Getränk insbesondere CO₂-haltig ist, wodurch - wie bereits beschrieben - die Qualität und der Geschmack des Getränks im Wesentlichen beeinflusst wird. Somit ergibt sich aus dem CO₂ Gehalt auch ein eindeutiger Rückschluss auf die Qualität und den Geschmack des Getränkes und das zu erreichende Mindesthaltbarkeitsdatum.

Bei dem erfindungsgemäßen Verfahren kann es optional vorgesehen sein, dass in einem Schritt o1) vor einer Promessung die Verpackung mit dem Lebensmittel zumindest geschüttelt oder auf eine vordefinierte Temperatur gebracht wird, um ein Gleichgewichtszustand innerhalb der Verpackung zu erreichen. Der Gleichgewichtszustand innerhalb der Verpackung ist für eine exakte Probenmessung sowohl bei der indirekten als auch bei der direkten Messung von wesentlicher Bedeutung. Ansonsten kann es zu gravierenden Messfehlern bei der Probenmessung (RMd,i bzw. Md,i) kommen, sodass insgesamt die Probenmessung unbrauchbar ist. Insbesondere bei CO₂-haltigen Getränken stellt sich ein einheitlicher Partialdruck in dem Gasraum und dem Flüssigkeitsraum der Verpackung durch das Schütteln ein.

Vorzugsweise findet bei dem erfindungsgemäßen Verfahren der zuvor beschriebene Schritt o1) des Schütteln der Probe zumindest mit oder zeitlich vor dem Schritt a1) oder b1) statt.

Damit kann erreicht werden, dass vor der jeweiligen Probenmessung gemäß Schritt a1) und/oder b1) ein Gleichgewichtszustand in der Verpackung erreicht wird und somit die Messergebnisse möglichst exakt sind. Auch kann die Verpackung mit dem Lebensmittel zuvor auf eine vordefinierte Temperatur gebracht werden, um somit temperaturbedingte Messfehler direkt auszuschließen. Dabei empfiehlt es sich, dass auch innerhalb der Verpackung eine homogene Temperatur vorhanden ist.

Des Weiteren kann es erfindungsgemäß vorgesehen sein, dass in einem Schritt o2) vor zumindest einer Probemessung wenigstens eine geometrische Eigenschaft der Verpackung messtechnisch erfasst wird. Bei der genannten geometrischen Eigenschaft, kann es sich z. B. um die äußeren Abmaße der Verpackung, insbesondere im Messbereich, beispielsweise dem Flaschenhals oder dem Kopfraum handeln. So kann z. B. der Außendurchmesser eines Getränkeflaschenhalses messtechnisch erfasst werden. Auch die Wandstärke der Verpackung im Messbereich kann technisch gemessen werden, um somit eine exakte Messung innerhalb der Verpackung vornehmen zu können. Hier kann z. B. der Innendurchmesser eines Flaschenhalses im Messbereich bestimmt werden, durch den z. B. die Lauflänge des Lichtstrahles bestimmbar ist. Aber auch der Brechungsindex des Verpackungsmaterials der Verpackung kann z. B. messtechnisch erfasst werden. Insgesamt dient die Erfassung der geometrischen Eigenschaften der Verpackung dazu, eine indirekte Probenmessung zu ermöglichen, bzw. Messfehler aufgrund von geometrischen Verpackungstoleranzen auszuschließen.

Bei dem erfindungsgemäßen Verfahren kann es auch vorgesehen sein, dass an derselben Verpackung zunächst eine indirekte und dann eine direkte Probenmessung RMi,d bzw. Mi,d zum Zeitpunkt Tx durchgeführt wird. Hierbei ist es denkbar, dass die indirekte Probenmessung RMi bzw. Mi kurz hintereinander mehrfach wiederholt wird, um beispielsweise einen statistischen Mittelwert bilden zu können. Nachdem die indirekte Probenmessung RMi bzw. Mi stattgefunden hat, kann dann an derselben Verpackung die direkte Probenmessung RMd bzw. Md erfolgen, die i. d. R. mit einer Zerstörung der Verpackung einhergeht. Auch diese direkte Probenmessung kann mehrfach wiederholt werden, um beispielsweise ebenfalls einen statistischen Mittelwert errechnen zu können. Dieses Vorgehen kann sowohl im Schritt a1), als auch in den Schritten b1) und b3) durchgeführt werden. Ebenfalls ist es denkbar, dass auch die indirekte und direkte Probenmessung gleichzeitig (gemeint ist simultan) an einer Probe stattfindet, wobei dabei aber sichergestellt werden muss, dass die direkte Probenmessung nicht zu einer Veränderung der physikalischen, chemischen und/oder biologischen Eigenschaften des Lebensmittels in der Verpackung führt. Somit muss vermieden werden, dass die direkte Probenmessung beendet ist, bevor die indirekte Probenmessung abgeschlossen worden ist.

Bei der Erfindung ist es ferner möglich, dass eine indirekte Probenmessung des Lebensmittels durch zumindest eine optische Messung durch die Verpackung stattfindet. Diese optische Messung muss nicht im sichtbaren Lichtbereich für Menschen stattfinden. So kann z. B. auch eine Messung im infraroten oder ultravioletten Strahlungsbereich stattfinden. Auch andere elektromagnetische Spektren des Lichtes sind dabei denkbar. Wie zuvor erwähnt worden ist, kann auch eine Messung durch Ultraschall oder Röntgenstrahlen stattfinden.

Des Weiteren ist es im Rahmen des erfindungsgemäßen Verfahrens denkbar, dass die Verpackung zumindest teilweise (licht-)durchsichtig ausgestaltet ist oder zumindest ein optisch durchsichtiges Messfenster aufweist. Dabei muss dieser optisch durchsichtige Bereich nicht für das menschliche Auge durchsichtig sein, sondern nur für die zuvor beschriebenen optischen Messungen durchdringbar sein, um somit möglichst ohne Messfehler auf optische Art und Weise die Eigenschaften des Lebensmittels in der Verpackung messtechnisch erfassen zu können. Es versteht sich von selbst, dass ein entsprechender optischer Sensor geometrisch im optisch durchsichtigen Bereich der Verpackung misst. Hierzu kann auf einer gegenüberliegenden Seite des optischen Sensors eine entsprechende Lichtquelle, welche die optische Strahlung aussendet, angeordnet sein. Selbstverständlich sind auch mehrere optische Sensoren sowie Lichtquellen zur Durchführung des erfindungsgemäßen Verfahrens denkbar, die auch nicht alle in dem gleichen Frequenzspektrum, bzw. der gleichen Wellenlänge funktionieren müssen. Im Gegenteil, durch Lichtquellen, die zumindest zeitweise unterschiedliches Licht von unterschiedlichen Wellenlängen aussendet, kann eine optische Messung erleichtert werden. Auch monochromatisches, polarisiertes und/oder gepulstes Licht usw. kann zur optischen Messung verwendet werden.

Bei dem erfindungsgemäßen Verfahren kann es zusätzlich denkbar sein, dass die Verpackung das Lebensmittel druckdicht aufnimmt und umschließt. Hierbei kann die Verpackung insbesondere als Flasche oder Flüssigkeitsbehälter ausgestaltet sein. Üblicherweise weist eine derartige Verpackung einen Deckel auf, durch den die Flüssigkeit, insbesondere in Form eines Getränkes, aus der Verpackung ausgeschüttet werden kann.

Ebenfalls ist die vorliegende Erfindung auch auf eine Vorrichtung zur Haltbarkeitsuntersuchung von Lebensmitteln in Verpackungen gemäß dem Anspruch 12 gerichtet. Dabei kann auf dieser Vorrichtung auch das zuvor erwähnte erfindungsgemäße Verfahren ausführbar sein.

Im Rahmen der erfindungsgemäßen Vorrichtung ist es ferner denkbar, dass die mechanische Aufnahme für die Verpackung gleichzeitig mit einem Dreh- und/oder Schwenkmechanismus ausgestaltet ist. Durch diesen Dreh- und/oder Schwenkmechanismus kann die Verpackung geschüttelt werden, um einen Gleichgewichtszustand innerhalb der Verpackung herzustellen und damit eine exakte Probenmessung zu erzielen.

Ferner kann es bei der erfindungsgemäßen Vorrichtung vorgesehen sein, dass die mechanische Aufnahme für die Verpackung gleichzeitig mit dem Dreh-und/oder Schwenkmechanismus ausgestaltet ist. Somit kann die Verpackung durch die mechanische Aufnahme sicher an dem Dreh- und/oder Schwenkmechanismus fixiert werden. Folglich wird die mechanische Aufnahme durch die von dem Dreh- oder Schwenkmechanismus mitgedreht, wodurch dann ebenfalls die Verpackung mit dem Lebensmittel bewegt wird. Zu diesem Zweck kann an der Vorrichtung ein entsprechender Antriebsmotor vorgesehen sein, der den Dreh- und/oder Schwenkmechanismus elektromechanisch antreibt.

Des Weiteren kann bei der erfindungsgemäßen Vorrichtung vorgesehen sein, dass der Meßkopf mit einem Anstechmittel versehen ist, wodurch eine direkte Messung mit zumindest einem Sensor in der Verpackung realisierbar ist. Idealerweise ist dieser Meßkopf ebenfalls an dem Dreh- und Schwenkmechanismus angeordnet und wird somit von diesem ebenfalls mit der Verpackung gedreht. Über das zuvor erwähnte Anstechmittel ist es möglich, einen Sensor innerhalb der Verpackung anzuordnen und damit eine direkte Messung zu ermöglichen. Idealerweise wird jedoch die Verpackung durch das Anstechmittel, bzw. den entsprechenden Meßkopf weiterhin abgedichtet, sodass z. B. ein Überdruck in der Verpackung nicht entweichen kann. Auch bei einer Flüssigkeit als Lebensmittel kann diese nicht über das abgedichtete Anstechmittel aus der Verpackung entweichen. Trotzdem ist es möglich, ein Meßsensor sogar bis in das Lebensmittel selbst einzuführen und somit die physikalischen, chemischen und/oder biologischen Eigenschaften direkt darin zu messen. Selbstverständlich können auch mehrere Eigenschaften des Lebensmittels messtechnisch erfasst werden, wobei mit einem oder mehreren Sensoren auch weitere Eigenschaften messbar sind. So kann z. B. die Temperatur und der Druck innerhalb des Lebensmittels gemessen werden. Z. B. lässt sich auch der elektrische Widerstand des Lebensmittels sowie weitere Eigenschaften auf einfache Art und Weise erfassen.

Bei der erfindungsgemäßen Vorrichtung ist es ferner denkbar, dass zumindest einer der nachfolgenden Sensoren vorhanden ist: Temperatursensor, Drucksensor, optischer Sensor, Gewichtssensor, Feuchtigkeitssensor, kapazitiver- oder induktiver Sensor, Widerstandssensor und dergleichen. Dieser Sensor kann in der Vorrichtung und/oder dem Meßkopf integriert sein. Ferner ist es denkbar, dass der Sensor über das Anstechmittel in die Verpackung einführbar ist. Auch können mehrere Sensoren in der Vorrichtung oder in dem Meßkopf der Vorrichtung angeordnet sein. Auch sogenannte Kombisensoren, die mehrere Eigenschaften messen können, können zur Anwendung kommen.

Im Rahmen der erfindungsgemäßen Vorrichtung ist es auch möglich, dass wenigstens eine Lichtquelle, insbesondere in Form eines Lasers vorhanden ist und dass das ausgestrahlte Licht von einer Lichtquelle von einem optischen Sensor messtechnisch erfassbar ist. Selbstverständlich können auch mehrere Lichtquellen in der Vorrichtung geometrisch zueinander angeordnet sein, um somit auch eine Messstrecke, bzw. ein Messfeld optisch messtechnisch zu erfassen. Als optische Sensoren können hier sogenannte Zeilensensoren oder Arraysensoren ausgestrahltes Licht der Lichtquelle messtechnisch erfassen. Eine Steuerung der Lichtquelle sowie eine Auswertung der erfassten messtechnischen Signale von dem oder den optischen Sensoren kann durch die Auswerteeinheit in der Vorrichtung stattfinden. Gleichzeitig können die erhaltenen optischen Messdaten mit Messdaten eines Sensors, der innerhalb der Verpackung angeordnet ist, verglichen, verarbeitet und/oder gespeichert werden. Selbstverständlich kann jedoch auch die optische Messung getrennt von der direkten Probemessung stattfinden.

Ferner ist es erfindungsgemäß denkbar, dass die Vorrichtung wenigstens eine Temperiereinheit zum Temperieren der Verpackung mit dem Lebensmittel aufweist. Dabei dient die Temperiereinheit dazu, die Verpackung mit dem Lebensmittel auf eine vordefinierte Temperatur zu bringen, sodass Messfehler aufgrund von unterschiedlichen Temperaturen verhindert werden. Außerdem ist es somit möglich, dass zumindest bei einer indirekten, zerstörungsfreien Probenmessung die Kühlung des Lebensmittels während der Messung nicht unterbrochen wird.

Weitere Maßnahmen und Vorteile der Erfindung ergeben sich aus den Ansprüchen, der nachfolgenden Beschreibung und den Zeichnungen. Ebenfalls gelten die offenbarten Merkmale aus der erfindungsgemäßen Vorrichtung auch für das erfindungsgemäße Verfahren und umgekehrt. In den Zeichnungen ist die Erfindung in einem schematischen Ausführungsbeispiel dargestellt.

Es zeigen:
- Fig. 1: schematische Ansicht einer erfindungsgemäßen Vorrichtung zur Haltbarkeitsuntersuchung von Lebensmitteln in Verpackungen und
- Fig. 2: beispielhaftes Diagramm zum Probenverbrauch nach dem Stand der Technik (NS) und nach dem erfindungsgemäßen Verfahren (NE)

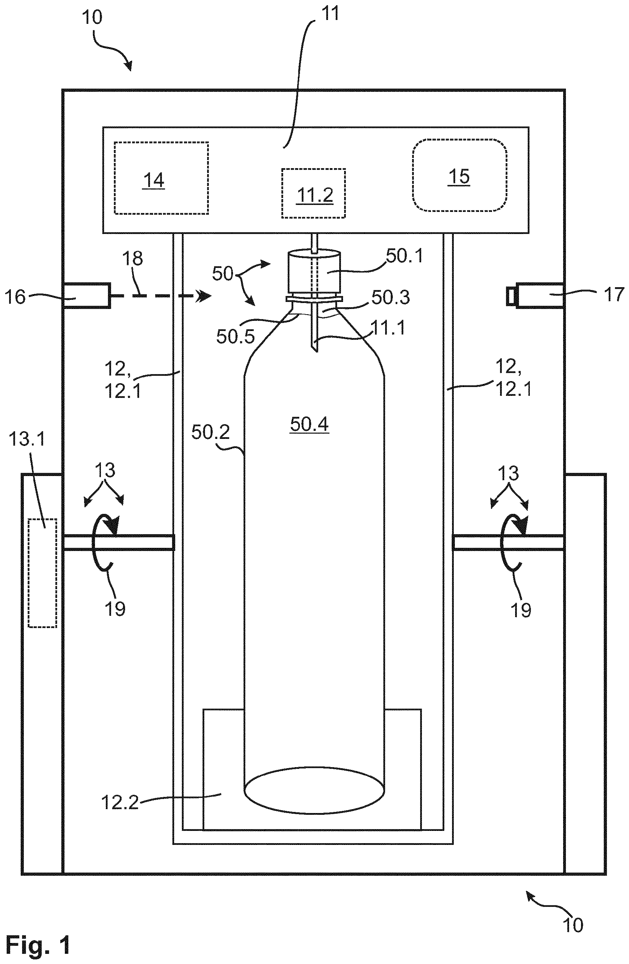

In der Figur 1 ist schematisch eine erfindungsgemäße Vorrichtung 10 zur Haltbarkeitsuntersuchung von Lebensmitteln in Verpackungen in Seitenansicht dargestellt. Dabei weist diese Vorrichtung 10 eine mechanische Aufnahme 12 für die zu messende Verpackung 50 mit dem entsprechenden Lebensmittel 50.4 auf. Bei der zu untersuchenden Verpackung 50 handelt es sich insbesondere um einen Flüssigkeitsbehälter in Form einer Flasche 50.2, die mit einem Deckel 50.1 verschlossen ist. Innerhalb der Flasche 50.2 ist das Lebensmittel 50.4 in Form eines Getränkes eingefüllt. Ein Füllstand 50.5 des Getränkes in der Flasche 50.2 ist ebenfalls schematisch angedeutet. Über dem Füllstand 50.5 befindet sich ein Kopfraum 50.3, der üblicherweise nicht mit dem Lebensmittel 50.4, sondern mit einem gasförmigen Medium, welches üblicherweise CO₂ haltig ist, gefüllt ist. Damit dieses Gas im Kopfraum 50.3 nicht entweichen kann, ist die Flasche 50.2 mit dem Deckel 50.1 verschlossen.

Wie ferner aus der Figur 1 hervorgeht, ist die Verpackung 50 durch ein Anstechmittel 11.1 angestochen worden, welches beispielsweise durch den Deckel 50.1 bis in das Getränk 50.4 ragt. Im Rahmen der Erfindung ist es jedoch ausreichend, wenn das Anstechmittel 11.1 durch den Deckel 50.1 bis in den Kopfraum 50.3 der Flasche 50.2 geführt wird. Das Anstechmittel 11.1 gehört selbst zum Meßkopf 11, der sich oberhalb des Deckels 50.1 befindet. Dabei kann der Meßkopf 11 auch zum Abdichten des Deckels 50.1 beim Anstechen durch das Anstechmittel 11.1 dienen, was im vorliegenden Fall jedoch nicht aus der Figur 1 - zum besseren Verständnis der Erfindung - hervorgeht. In dem Meßkopf 11, der zur erfindungsgemäßen Vorrichtung 10 gehört, kann zumindest ein Sensor 11.2 angeordnet sein. Bei diesem Sensor 11.2 kann es sich insbesondere um einen Temperatursensor und/oder Drucksensor handeln. Auch können mehrere Sensoren 11.2 in dem Meßkopf 11 vorgesehen sein, die ebenfalls die Eigenschaften des Lebensmittels 50.4 durch das Anstechmittel 11.1 direkt messtechnisch erfassen. Des Weiteren ist die Vorrichtung 10 mit einem Dreh- und oder Schwenkmechanismus 13 ausgestattet, der mehr oder weniger bügelartig 12.1 ausgestaltet ist und die Verpackung 50 sicher über die mechanische Aufnahme 12 aufnimmt, bzw. fixiert. Am unteren Ende des Bügels 12.1 der mechanischen Aufnahme 12 ist eine Halterung 12.2 angeordnet, die zur formschlüssigen und/oder kraftschlüssigen Halterung der Verpackung 50 dient. Durch diese mechanische Aufnahme 12 kann auch die Verpackung 50 geometrisch exakt innerhalb der Messvorrichtung 10 justiert werden. Der Meßkopf 11, der ebenfalls an dem Dreh- und/oder Schwenkmechanismus 13 mit dem Bügel12.1 der mechanischen Aufnahme 12 verbunden ist, kann zusätzlich über eine Auswerteeinheit 14 sowie eine Anzeige 15 verfügen. Auch ist es denkbar, dass zumindest eine Auswerteeinheit 14 oder eine entsprechende Anzeige 15 stationär, d. h. unabhängig von dem Dreh- und Schwenkmechanismus innerhalb der Vorrichtung 10 angeordnet ist.

Anstelle des Bügel 12.1 kann auch ein geschlossenes Gehäuse als mechanische Aufnahme 12 dienen, innerhalb dessen z. B. auch die zuvor beschriebene Temperiereinheit für die Verpackung 50 anordbar ist. Diese Temperiereinheit ist jedoch in der Figur 1 nicht dargestellt.

Des Weiteren kann innerhalb der Vorrichtung 10, insbesondere im Bereich des Kopfraumes 50.3 der Flasche 50.2 zumindest eine Lichtquelle 16, z. B. in Form eines Lasers, angeordnet sein, in die ein Lichtstrahl 18 aussendet, der durch die Verpackung 50 stahlt. Von der von der Lichtquelle 16 gegenüberliegenden Seite der Verpackung 50 kann ein optischer Sensor 17 innerhalb der Vorrichtung 10 angeordnet sein, der den ausgesendeten Lichtstrahl 18 messtechnisch erfasst. Auch kann an der Lichtquelle 16 selbst ein optischer Sensor 17 vorgesehen sein, der einen Teil des reflektierten Lichtstahles 18 der Lichtquelle 16 misst. Durch die vorgesehene Lichtquelle 16 sowie den optischen Sensor 17 lässt sich eine zerstörungsfreie, indirekte Probenmessung RMi und Mi der Verpackung 50 mit dem Lebensmittel 50.4 vornehmen. Wie bereits erwähnt, ist der ausgesendete Lichtstrahl 18 nicht auf das für den Menschen sichtbare Licht beschränkt. Ferner sind auch Lichtstrahlen 18 einer anderen Wellenlänge denkbar.

Um eine optimale Probenvorbereitung zu erreichen, ist die Vorrichtung 10 mit dem bereits erwähnten Dreh- und/oder Schwenkmechanismus 13 ausgestattet. Dieser wird durch einen elektromechanischen Antrieb 13.1 angetrieben, der beispielsweise durch einen E-Motor realisiert werden kann. Der Dreh- und oder Schwenkmechanismus 13 dreht dabei die mechanische Aufnahme 12 mit der fixierten Verpackung 50 und dem an der mechanischen Aufnahme 12 angeordneten Meßkopf 11. Auch ist es denkbar, dass zumindest eine Lichtquelle 16 und/oder ein optischer Sensor 17 an der mechanischen Aufnahme 12 angeordnet ist und nicht stationär mit der Vorrichtung 10 verbunden ist, wie in der Figur 1 dargestellt.

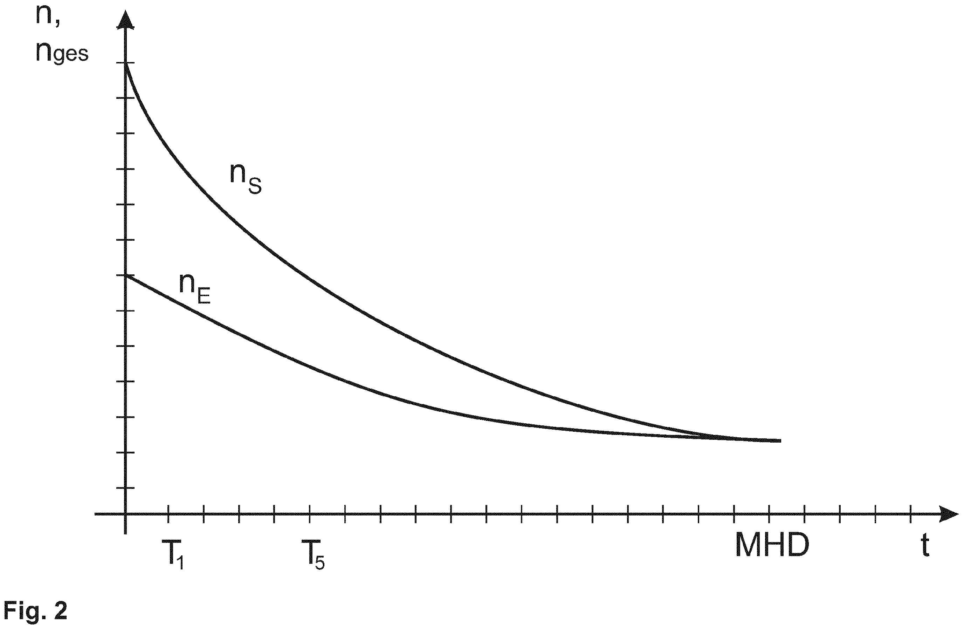

In der Figur 2 ist rein schematisch ein Vergleich der reduzierten Probenanzahl n_{E} des erfindungsgemäßen Verfahrens zum Vergleich der Probenanzahl ns aus dem Stand der Technik in einem Diagramm dargestellt. Dabei wird deutlich, dass sich die Probenanzahl n_{E} durch das erfindungsgemäße Verfahren deutlich, vorzugsweise halbieren bzw. vierteln und noch weiter reduzieren lässt, woraus sich die bereits beschriebenen Vorteile der Erfindung ergeben.

### Bezugszeichenliste

- 10: Vorrichtung
- 11: Meßkopf
- 11.1: Anstechmittel
- 11.2: Sensor, insbesondere Temperatur- / Druck- / und Feuchtigkeitssensor
- 12: mechanische Aufnahme für 50
- 12.1: Bügel mit Aufnahme für 50
- 12.2: Halterung für 50
- 13: Dreh- und/oder Schwenkmechanismus
- 13.1: Antrieb
- 14: Auswerteeinheit
- 15: Anzeige
- 16: Lichtquelle, insbesondere Laser
- 17: Optischer Sensor für 16
- 18: Pfeil für Lichtstrahl
- 19: Pfeil für Drehrichtung

- 50: Verpackung
- 50.1: Deckel
- 50.2: Flasche, Flüssigkeitsbehälter
- 50.3: Kopfraum
- 50.4: Lebensmittel, insbesondere Getränk
- 50.5: Füllstand

## Patentansprüche

1. Verfahren zu Haltbarkeitsuntersuchungen von Lebensmitteln (50.4) in Verpackungen (50),
**dadurch gekennzeichnet,**
**dass** die folgenden Schritte durchgeführt werden:
a) Erstellen zumindest einer **Referenzmessung** mit:
a1) einer direkten und indirekten Probenmessung RMd,i zum Zeitpunkt Tx, wobei eine direkte Probenmessung des Lebensmittels durch Zerstörung der Verpackung stattfindet und eine indirekte Probenmessung des Lebensmittels durch eine zerstörungsfreie Untersuchung der Verpackung stattfindet,
a2) Wiederholung der direkten und indirekten Probenmessung RMd,i in vordefinierten Zeitabständen Δt
a3) Speicherung der Referenzmeßergebnisse RMd,i der Probenmessungen
b) Erstellen einer **Vergleichsmessung** mit:
b1) zumindest einer indirekten Probenmessung Mi zum Zeitpunkt Tx
b2) Vergleich des indirekten Meßergebnisses Mi mit dem entsprechenden Referenzmeßergebnis RMi vom Vergleichszeitpunkt Tx
b3) Durchführung von zumindest einer direkten Probenmessung Md zum Zeitpunkt Tx bei einer überschrittenen Abweichung des Vergleichsergebnisses aus Schritt b2)
b4) Wiederholung der indirekten Probenmessung in vordefinierten Zeitabständen Δt
b5) Speicherung der Meßergebnisse Md,i der Probenmessungen.

2. Verfahren nach Anspruch 1,
**dadurch gekennzeichnet,**
**dass** bei einer direkten Probenmessung des Lebensmittels (50.4) insbesondere die Verpackung (50) angestochen wird, um wenigstens einen Meßsensor (11.2) in die Verpackung (50) einzuführen, um zumindest eine physikalische, chemische und/oder biologische Eigenschaft des Lebensmittels (50.4) zu messen.

3. Verfahren nach Anspruch 1 oder 2,
**dadurch gekennzeichnet,**
**dass** bei einer indirekten Probenmessung des Lebensmittels (50.4) insbesondere physikalische, chemische und/oder biologische Eigenschaften des Lebensmittels (50.4) vorzugsweise kontaktlos durch die Verpackung (50) gemessen werden.

4. Verfahren nach einem der vorhergehenden Ansprüche,
**dadurch gekennzeichnet,**
**dass** bei der Probenmessung zumindest die Temperatur oder der Druck gemessen wird, und/oder dass ein CO2-Gehalt in der Verpackung (50) aus den Messwerten RMd, i/Md,i der Temperatur und des Druckes insbesondere über eine Berechnungsfunktion ermittelt wird.

5. Verfahren nach einem der vorhergehenden Ansprüche,
**dadurch gekennzeichnet,**
**dass** in einem Schritt:
o1) vor einer Probenmessung die Verpackung (50) mit dem Lebensmittel (50.4) zumindest geschüttelt oder auf eine vordefinierte Temperatur gebracht wird, um einen Gleichgewichtszustand zu erreichen und/oder dass simultan zu Schritt o1) zumindest Schritt a1) oder b1) durchgeführt wird.

6. Verfahren nach einem der vorhergehenden Ansprüche,
**dadurch gekennzeichnet,**
**dass** in einem Schritt:
o2) vor zumindest einer Probenmessung wenigstens eine geometrische Eigenschaft der Verpackung (50) messtechnisch erfasst wird.

7. Verfahren nach einem der vorhergehenden Ansprüche,
**dadurch gekennzeichnet,**
**dass** an derselben Verpackung (50) zunächst eine indirekte und dann eine direkte Probenmessung RMd, i bzw. Md,i zum Zeitpunkt Tx durchgeführt wird.

8. Verfahren nach einem der vorhergehenden Ansprüche,
**dadurch gekennzeichnet,**
**dass** eine indirekte Probenmessung des Lebensmittels (50.4) durch zumindest eine optische Messung durch die Verpackung (50) stattfindet.

9. Verfahren nach einem der vorhergehenden Ansprüche,
**dadurch gekennzeichnet,**
**dass** es sich bei dem Lebensmittel (50.4) um ein Getränk, insbesondere CO₂ haltiges Getränk handelt
und/oder dass der CO₂ Gehalt zumindest in der Verpackung (50) oder im Lebensmittel (50.4) die Haltbarkeit wesentlich beeinflusst.

10. Verfahren nach einem der vorhergehenden Ansprüche,
**dadurch gekennzeichnet,**
**dass** die Verpackung (50) zumindest teilweise optisch durchsichtig ausgestaltet ist oder zumindest ein optisch durchsichtiges Messfenster aufweist.

11. Verfahren nach einem der vorhergehenden Ansprüche,
**dadurch gekennzeichnet,**
**dass** die Verpackung (50) das Lebensmittel (50.4) druckdicht aufnimmt und umschließt, wobei insbesondere die Verpackung (50) als Flasche (50.2) oder Flüssigkeitsbehälter ausgestaltet ist.

12. Vorrichtung (10) zu Haltbarkeitsuntersuchungen von Lebensmitteln (50.4) in Verpackungen (50) mit einer mechanischen Aufnahme (12) für die Verpackung (50) und einem Meßkopf (11) und einer Auswerteeinheit (14),
**dadurch gekennzeichnet,**
**dass** die Vorrichtung (10) dazu eingerichtet ist, das Verfahren gemäß einem der vorherigen Ansprüche auszuführen.

13. Vorrichtung (10) nach einem der vorhergehenden Ansprüche,
**dadurch gekennzeichnet,**
**dass** die mechanische Aufnahme (12) für die Verpackung (50) gleichzeitig mit einem Dreh- und/oder Schwenkmechanismus (13) ausgestaltet ist
und/oder dass der Meßkopf (11) mit einem Anstechmittel (11.1) versehen ist, wodurch eine direkte Messung mit zumindest einem Sensor (11.2) in der Verpackung (50) realisierbar ist.

14. Vorrichtung (10) nach einem der vorhergehenden Ansprüche,
**dadurch gekennzeichnet,**
**dass** zumindest bei der Vorrichtung (10) oder in dem Meßkopf (11) zumindest einer der nachfolgenden Sensoren (11.2) vorhanden ist:
Temperatursensor, Drucksensor, optischer Sensor, Gewichtssensor, Feuchtigkeitssensor, Piezosensor, kapazitiver und/oder induktiver Sensor.

15. Vorrichtung (10) nach einem der vorhergehenden Ansprüche,
**dadurch gekennzeichnet,**
**dass** wenigstens eine Lichtquelle (16), insbesondere in Form eines Lasers vorhanden ist und das ausgestrahlte Licht (18) durch einen optischen Sensor (18) meßtechnisch erfassbar ist
und/oder dass wenigstens eine Temperiereinheit zum Temperieren der Verpackung (50) mit dem Lebensmittel (50.4) vorhanden ist.

## Claims

1. Method for shelf-life investigations of foodstuffs (50.4) in packagings (50), **characterized in**
**that** the following steps are performed:
a) create at least one **reference measurement,** including:
(a1) a direct and indirect sample measurement RMd,i at time Tx, where a direct sample measurement of the foodstuff takes place by destruction of the packaging and an indirect sample measurement of the food takes place by non-destructive examination of the packaging,
a2) repetition of direct and indirect sample measurement RMd,i at predefined intervals Δt
a3) storage of the reference measurement results RMd,i of the sample measurements
b) create a **comparison measurement** with:
b1) at least one indirect sample measurement Mi at time Tx
b2) comparison of the indirect measurement result Mi with the corresponding reference measurement result RMi from the comparison time Tx
b3) performance of at least one direct sample measurement Md at time Tx in the event of an exceeded deviation of the comparison result from step b2)
b4) repetition of the indirect sample measurement at predefined intervals Δt
b5) storage of the measurement results Md,i of the sample measurements.

2. Method according to claim 1,
**characterized in**
**that**, during a direct sample measurement of the foodstuff (50.4), in particular the packaging (50) is pierced in order to introduce at least one measuring sensor (11.2) into the packaging (50) in order to measure at least one physical, chemical and/or biological property of the foodstuff (50.4).

3. Method according to claim 1 or 2,
**characterized in**
**that** in an indirect sample measurement of the foodstuff (50.4), in particular physical, chemical and/or biological properties of the foodstuff (50.4) are preferably measured contactlessly through the packaging (50).

4. Method according to one of the preceding claims,
**characterized in**
**that** at least the temperature or the pressure is measured during the sample measurement,
and/or in that a CO₂ content in the packaging (50) is determined from the measured values RMd, i/Md,i of the temperature and the pressure, in particular via a calculation function.

5. Method according to one of the preceding claims,
**characterized in**
**that** in one step:
o1) prior to a sample measurement, the packaging (50) with the foodstuff (50.4) is at least shaken or brought to a predefined temperature in order to achieve a state of equilibrium
and/or in that at least step a1) or b1) is carried out simultaneously with step o1).

6. Method according to one of the preceding claims,
**characterized in**
**that** in one step:
o2) at least one geometric property of the packaging (50) is metrologically detected before at least one sample measurement.

7. Method according to one of the preceding claims,
**characterized in**
**that** first an indirect and then a direct sample measurement RMd, i or Md,i is carried out on the same packaging (50) at the time Tx.

8. Method according to one of the preceding claims,
**characterized in**
**that** an indirect sample measurement of the foodstuff (50.4) takes place by at least one optical measurement through the packaging (50).

9. Method according to one of the preceding claims,
**characterized in**
**that** the foodstuff (50.4) is a drink, in particular a drink containing CO₂ and/or that the CO₂ content at least in the packaging (50) or in the foodstuff (50.4) substantially influences the shelf life.

10. Method according to one of the preceding claims,
**characterized in**
**that** the packaging (50) is at least partially optically transparent or has at least one optically transparent measuring window.

11. Method according to one of the preceding claims,
**characterized in**
**that** the packaging (50) accommodates and encloses the foodstuff (50.4) in a pressure-tight manner,
in particular the packaging (50) being designed as a bottle (50.2) or liquid container.

12. Device (10) for testing the shelf life of foodstuffs (50.4) in packagings (50), having a mechanical receptacle (12) for the package (50) and a measuring head (11) and an evaluation unit (14),
**characterized in**
**that** the apparatus (10) is adapted to execute the method according to one of the preceding claims.

13. Device (10) according to any one of the preceding claims,
**characterized in**
**that** the mechanical receptacle (12) for the packaging (50) is configured simultaneously with a rotary and/or swivel mechanism (13)
and/or that the measuring head (11) is provided with a piercing means (11.1), whereby a direct measurement with at least one sensor (11.2) in the packaging (50) can be realised.

14. Device (10) according to any one of the preceding claims,
**characterized in**
**that** at least one of the following sensors (11.2) is present at least in the device (10) or in the measuring head (11):
Temperature sensor, pressure sensor, optical sensor, weight sensor, humidity sensor, piezo sensor, capacitive and/or inductive sensor.

15. Device (10) according to any one of the preceding claims,
**characterized in**
**that** at least one light source (16) is present, in particular in the form of a laser, and the emitted light (18) can be detected metrologically by an optical sensor (18)
and/or in that at least one tempering unit for tempering the packaging (50) with the foodstuff (50.4) is present.

## Revendications

1. Procédé d'analyse de la durée de conservation des aliments (50.4) dans les emballages (50),
**caractérise en ce**
**que** les étapes suivantes sont effectuées :
a) créer au moins une **mesure de référence :**
a1) une mesure directe et indirecte de l'échantillon RMd,i au temps Tx, où une mesure directe de l'échantillon de l'aliment est effectuée par destruction de l'emballage et une mesure indirecte de l'échantillon de l'aliment est effectuée par une analyse non destructif de l'emballage,
a2) répétition de la mesure directe et indirecte de l'échantillon RMd,i à des intervalles prédéfinis Δt
a3) mémorisation des résultats des mesures de référence RMd,i des mesures d'échantillons
b) créer une **mesure comparative** avec :
b1) au moins une mesure indirecte de l'échantillon Mi au temps Tx
b2) comparaison du résultat de mesure indirect Mi avec le résultat de mesure de référence correspondant RMi du temps de comparaison Tx
b3) réalisation d'au moins une mesure directe de l'échantillon Md à l'instant Tx en cas de dépassement de l'écart du résultat de la comparaison par rapport à l'étape b2)
b4) répéter la mesure indirecte de l'échantillon à des intervalles prédéfinis Δt
b5) mémorisation des résultats de mesure Md,i des mesures de l'échantillon.

2. Procédé selon la revendication 1,
**caractérise en ce**
**que**, lors d'une mesure directe de l'échantillon de l'aliment (50.4), en particulier l'emballage (50) est percé pour introduire au moins un capteur de mesure (11.2) dans l'emballage (50) afin de mesurer au moins une propriété physique, chimique et/ou biologique de l'aliment (50.4).

3. Procédé selon la revendication 1 ou 2,
**caractérise en ce**
**que**, dans une mesure de l'échantillon indirect de l'aliment (50.4), en particulier les propriétés physiques, chimiques et/ou biologiques de l'aliment (50.4) sont mesurées de préférence sans contact à travers l'emballage (50).

4. Procédé selon l'une des revendications précédentes,
**caractérise en ce**
**qu'**au moins la température ou la pression est mesurée pendant la mesure de l'échantillon,
et/ou **en ce qu'**une teneur en CO₂ dans l'emballage (50) est déterminée à partir des valeurs mesurées RMd, i/Md,i de la température et de la pression, en particulier par une fonction de calcul.

5. Procédé selon l'une des revendications précédentes,
**caractérise en ce**
**qu'**en une étape :
o1) avant la mesure de l'échantillon, l'emballage (50) contenant l'aliment (50.4) est au moins agité ou porté à une température prédéfinie afin d'atteindre un état d'équilibre et/ou **en ce qu'**au moins l'étape a1) ou b1) est exécutée simultanément avec l'étape o1).

6. Procédé selon l'une des revendications précédentes,
**caractérise en ce**
**qu'**en une étape :
o2) au moins une propriété géométrique de l'emballage (50) est détectée métrologiquement avant au moins une mesure d'échantillon.

7. Procédé selon l'une des revendications précédentes,
**caractérise en ce**
**que**, d'abord une mesure indirecte puis une mesure directe d'échantillon RMd, i ou Md,i sur le même emballages (50) au moment Tx sont effectuées.

8. Procédé selon l'une des revendications précédentes,
**caractérise en ce**
**qu'**une mesure indirecte de l'échantillon de l'aliment (50.4) est effectuée par au moins une mesure optique à travers l'emballage (50).

9. Procédé selon l'une des revendications précédentes,
**caractérise en ce**
**que** l'aliment (50.4) est une boisson, en particulier une boisson contenant du CO₂ et/ou que la teneur en CO₂ au moins dans l'emballage (50) ou dans l'aliment (50.4) influence sensiblement la durée de conservation.

10. Procédé selon l'une des revendications précédentes,
**caractérise en ce**
**que** l'emballage (50) est au moins partiellement transparent optiquement ou présente au moins une fenêtre de mesure optiquement transparente.

11. Procédé selon l'une des revendications précédentes,
**caractérise en ce**
**que** l'emballage (50) reçoit et enferme l'aliment (50.4) de manière étanche à la pression, en particulier l'emballage (50) étant conçu comme une bouteille (50.2) ou un récipient pour liquide.

12. Dispositif (10) pour contrôler la durée de conservation d'aliments (50.4) dans des emballages (50), comprenant un récipient mécanique (12) pour l'emballage (50) et une tête de mesure (11) et une unité d'évaluation (14),
**caractérise en ce**
**que** le dispositif (10) est adapté pour exécuter le procédé selon l'une des revendications précédentes.

13. Dispositif (10) selon l'une des revendications précédentes,
**caractérise en ce**
**que** le récipient mécanique (12) pour l'emballage (50) est configuré simultanément avec un mécanisme rotatif et/ou pivotant (13)
et/ou **en ce que** la tête de mesure (11) est munie d'un moyen de perçage (11.1), permettant de réaliser une mesure directe avec au moins un capteur (11.2) dans l'emballage (50).

14. Dispositif (10) selon l'une des revendications précédentes,
**caractérise en ce**
**qu'**au moins un des capteurs suivants (11.2) est présent au moins dans le dispositif (10) ou dans la tête de mesure (11) :
capteur de température, capteur de pression, capteur optique, capteur de poids, capteur d'humidité, capteur piézoélectrique, capteur capacitif et/ou inductif.

15. Dispositif (10) selon l'une des revendications précédentes,
**caractérise en ce**
**qu'**au moins une source lumineuse (16) est présente, en particulier sous la forme d'un laser, et la lumière émise (18) peut être détectée métrologiquement par un capteur optique (18)
et/ou **en ce qu'**il existe au moins une unité de trempe pour la trempe de l'emballage (50) avec l'aliment (50.4).
